(19) Europäisches Patentamt / European Patent Office / Office européen des brevets

(11) **EP 2 854 875 B1**

(12) **EUROPEAN PATENT SPECIFICATION**

(45) Date of publication and mention
of the grant of the patent:
**29.03.2017 Bulletin 2017/13**

(51) Int Cl.:
*A61L 2/24* *(2006.01)*  *G01K 13/02* *(2006.01)*
*G01K 1/02* *(2006.01)*

(21) Application number: **12877982.4**

(22) Date of filing: **31.05.2012**

(86) International application number:
**PCT/CN2012/076334**

(87) International publication number:
**WO 2013/177777 (05.12.2013 Gazette 2013/49)**

(54) **AN ELECTRONIC INDICATOR DEVICE FOR CLEANING MONITORING**

ELEKTRONISCHER INDIKATOR ZUR REINIGUNGSÜBERWACHUNG

DISPOSITIF INDICATEUR ÉLECTRONIQUE POUR SURVEILLANCE DE NETTOYAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.04.2015 Bulletin 2015/15**

(73) Proprietor: **3M Innovative Properties Company
St. Paul, Minnesota 55144 (US)**

(72) Inventors:
• **ZHOU, Pingle**
**Shanghai 200333 (CN)**
• **YANG, Yinghua**
**Shanghai 200336 (CN)**
• **ZHANG, Chunyu**
**Shanghai 200336 (CN)**
• **WEN, Zhiguo**
**Shanghai 200336 (CN)**
• **LIU, Dong**
**Shanghai 200336 (CN)**

(74) Representative: **Vossius & Partner
Patentanwälte Rechtsanwälte mbB
Siebertstrasse 3
81675 München (DE)**

(56) References cited:
EP-A1- 0 604 387    EP-A2- 1 308 718
WO-A1-98/36781    WO-A1-2006/116835
WO-A2-99/32159    WO-A2-2007/001866
CN-A- 1 202 831    CN-A- 1 279 113
US-A- 4 539 929    US-A1- 2003 171 943

## Description

### Technical Field

The present disclosure relates to electronic devices used for monitoring cleaning cycles. This disclosure relates to monitoring devices that can be disposed in a wash chamber of a washer-disinfector to monitor the efficacy of a cleaning cycle.

### Background art

WO 2007/001 866 A2 discloses an apparatus comprising an electronic sensor configured to be carried by a person and to be used by the person to detect a cleanliness state of the person's hands.

EP0604387 A1 discloses a measurement system for use with an autoclave, and the like, has a thermally insulated container with two Dewar flasks where one is within the other, with opposing alignment. The inner Dewar flask contains the electronic measurement system, and the connecting lines pass between the flasks. Couplings are fitted tightly to the cover with matching couplings for connection to the transmission aerial and the link to at least one sensor. The electronic measurement system has an analogue/ digital converter to change the measurement data into digital form, a digital control and processor component, and a following transmission unit.

EP1308718 A2 discloses an oxidative gas/vapor concentration monitoring apparatus, for sterilization of surgical instruments, generates net voltage corresponding to gas/vapor concentration across thermocouple junctions, upon chemical substance exposure.

WO 98/36 781 A1 discloses a remote sterilization indicator is described. The indicator allows a sterilization cycle to be monitored without the need to visually inspect the indicator.

### Summary

The invention is defined by independent claims 1 and 10.

At least one aspect of the present disclosure features an apparatus for determining the efficacy of a cleaning cycle that is configured to be placed in a wash chamber containing a fluid, the cleaning cycle comprising a wash cycle and a thermal disinfection cycle, the apparatus comprising a housing, a thermal sensor, and a processor disposed in the housing and communicatively coupled to the thermal sensor. The exterior of the housing is configured to be in contact with the fluid. The thermal sensor is configured to provide signals indicative of temperature of the fluid and at least part of the thermal sensor is disposed in the housing. The processor is configured to receive the signals from the thermal sensor. Based on the received signals, the processor is configured to: determine an efficacy of the wash cycle; determine an efficacy of the thermal disinfection cycle; and determine the efficacy of the cleaning cycle based on the efficacy of the wash cycle and the efficacy of the thermal disinfection cycle.

At least one aspect of the present disclosure features a method of evaluating an efficacy of a cleaning cycle comprising a wash cycle and a thermal disinfection cycle, the method including the steps of: receiving temperature data and time data collected during the wash cycle; selecting, by a processor, a first time period within the wash cycle during which the temperature data is indicative of temperatures exceeding a wash threshold temperature; determining, by the processor, a wash efficacy based on the temperature data collected during the first time period; receiving temperature data and time data collected during the thermal disinfection cycle; selecting a second time period within the thermal disinfection cycle during which the temperature data is indicative of temperatures exceeding a disinfection temperature threshold; determining, by the processor, a disinfection efficacy based on the temperature data collected during the second time period; and determining, by the processor, the efficacy of the cleaning cycle based on the wash efficacy and the disinfection efficacy.

### Brief Description of the Drawings

The accompanying drawings are incorporated in and constitute a part of this specification and, together with the description, explain the advantages and principles of the invention. In the drawings,

Figure 1 is a graph illustrating a relationship between enzyme activity and temperature;
Figure 2A illustrates a block diagram of an embodiment of an electronic indicator device;
Figure 2B illustrates an exemplary system diagram of an electronic indicator device;
Figure 2C illustrates some exemplary functional modules that may be included in a processing unit;
Figure 3A illustrates an exemplary flow diagram of an electronic indicator device;
Figure 3B illustrates an exemplary flow diagram for determining an efficacy of a wash cycle;

Figure 3C illustrates an exemplary flow diagram for determining an efficacy of a thermal disinfection cycle;

Figure 4 is a graph illustrating a time-temperature curve corresponding to a cleaning cycle;

Figure 5A is an exploded view of an exemplary embodiment of an electronic indicator device;

Figure 5B is a perspective view of an embodiment of an electronic indicator device;

Figures 6A, 6B, and 6C illustrate a cross-sectional view of an exemplary embodiment of a switch used by an electronic indicator device;

Figures 7A, 7B, 7C illustrate electronic indicator devices with labels;

Figure 8 illustrates a system diagram of an exemplary embodiment of a cleaning monitoring system; and

Figures 9A and 9B illustrate two graphs of temperature data collected by the electronic indicator device within two cleaning cycles.

## Detailed Description

Decontamination is a process of cleaning objects using physical and/or chemical means. Hospital or other health care facilities often use heat, either steam or hot water, to decontaminate medical devices. Additionally, washing in hot water at proper temperature and for a sufficient amount of time provides a broad disinfecting effect. Disinfection is a process capable of destroying and/or removing pathogenic microorganisms. Disinfection processes are intended to destroy or prevent growth of microorganisms capable of causing infections. The microorganisms include, for example, vegetative bacteria, pathogenic fungi, and specifically tested viruses. Disinfection process can be a thermal disinfection process, a chemical disinfection process using chemical disinfectant(s), or a combination thereof. Washing in hot water is a popular way for thermal disinfection due to its low cost and personal and environmental safety.

Reusable devices or articles often require wash and disinfection after each use. It is important to monitor and ensure the efficacy of such wash and disinfection in cleaning theses devices or articles, especially in cleaning devices or articles to be used in medical, dental, pharmaceutical and veterinary practices. Fluid, such as hot water or steam, is often used in the wash and thermal disinfection process. During a wash process, cleaning agents and enzymes may also be used in the hot water. An effective wash process may require the fluid to be heated to an appropriate temperature range to activate the cleaning agents and enzymes for a sufficiently long time period. A thermal disinfection process may require the fluid to be heated to a minimum temperature and for an adequate time period. For example, in ISO 15883-1, "A" value, a value of equivalent time in seconds at 80°C, is used to evaluate a disinfection efficacy.

Aspects of this disclosure are directed to embodiments of a monitoring device capable of measuring environmental conditions within a washer-disinfector chamber, collecting environmental data indicative of the environmental conditions during a cleaning cycle, and determining efficacy of a cleaning cycle based on the collected environmental data. In some cases, a cleaning cycle includes a sequence of wash cycle, rinse cycle, thermal disinfection cycle, and drying cycle. In some embodiments, the efficacy of a cleaning cycle can be determined by a three- step computation: 1) determine an efficacy of the wash cycle, also referred to as a wash efficacy, based on the temperature collected during the wash cycle; 2) determine an efficacy of the thermal disinfection cycle, also referred to as a disinfection efficacy based on the temperature collected during the disinfection cycle; and 3) determine the efficacy of the cleaning cycle based on both the wash efficacy and the disinfection efficacy.

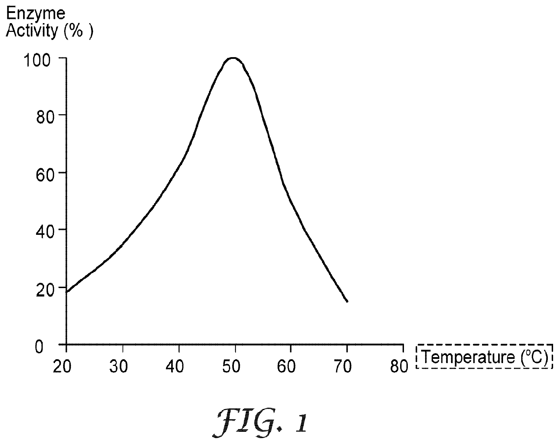

Cleaning enzymes (i.e., proteases, lipases, amylases, etc.) are activated in a suitable wash temperature range during the wash cycle, where the range depends on the particular enzyme used. Further, the level of activeness of a cleaning enzyme typically has a linear relationship with the temperature within the suitable wash temperature range, so the effectiveness of the cleaning enzyme can be indicated by the heat equivalence during the wash cycle. For example, as illustrated in Figure 1, the particular enzyme is more active when the temperature is higher (i.e. the activity of the enzyme is greater when the temperature is higher) in the temperature range of 30°C-50°C.

Aspects of this disclosure are also directed to an electronic indicator device with waterproof, air-pressure withstood, and adequate heat insulation design, which can be placed within a washer-disinfector chamber to measure environmental conditions and collect environmental data indicative of the environmental conditions during a cleaning cycle. The portable device can further determine the efficacy of the cleaning cycle based on the collected environment data and optionally present the efficacy of the cleaning cycle via an indicator that is part of the device. For example, an indicator can be a group of light emitting diodes (LEDs) with a light guide channeling the LED light to the outer cover of the electronic indicator device, where a green light indicates "Pass" (i.e., effective) or a red light indicates "Fail" (i.e., ineffective).

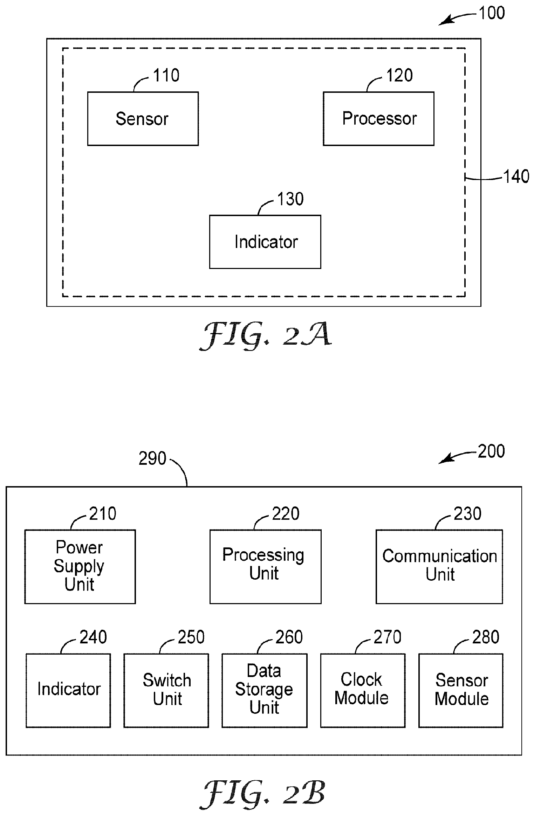

To facilitate the understanding of the present disclosure, Figure 2A illustrates a block diagram of an embodiment of an electronic indicator device 100. The device 100 includes one or more environmental sensors 110, a processor 120, and an optional indicator 130. An environmental sensor 110 can detect environmental conditions and generate signals corresponding to the environmental conditions. In some implementations, the one or more environmental sensors 110 include a thermal sensor that can detect temperature of surrounding environments. The processor 120 can be communicatively coupled to the one or more sensors 110 and receive the signals generated by the sensors 110. In some embodiments, the device 100 has a housing 140, where the processor 120 and at least part of the indicator 130 can be disposed in the housing 140. In some cases, the indicator 130 can be visible through a transparent part of the housing 140.

In some implementations, the device 100 is configured to be placed in a wash chamber containing a fluid and the exterior of the housing 140 configured to be in contact with the fluid during a cleaning cycle. The fluid can be hot water, hot water with cleaning enzyme, steam or other types of fluid used in a cleaning cycle. In some cases, the fluid can be changed during the cleaning cycle. For example, the fluid can be hot water with cleaning enzyme in a wash cycle and then change to hot water without any chemical substances in a thermal disinfection cycle. In such cases, the fluid is referred to the different type of fluid in general used in the wash chamber during a cleaning cycle. In some configurations, the housing 140 provides waterproof and heat insulation to ensure adequate environment for the processor 120. The processor 120 may comprise one or more microprocessors, digital signal processors, processors, PICs (Programmable Interface Controllers), microcontrollers, or any other form of computing devices.

In some embodiments, the one or more environmental sensors 110 are configured to provide signals indicative of temperature of the fluid during a cleaning cycle. In some implementations, an environmental sensor 110 is disposed in the housing 140. In some other implementations, the environmental sensor 110 is disposed outside the housing 140 but communicatively coupled to the processor 120 disposed in the housing 140. In yet other implementations, the housing 140 includes two parts: an inner housing and an outer housing, where the environmental sensor 110 is disposed outside of the inner housing and at least part of the environmental sensor 110 is disposed within the outer housing. In some cases, the processor 120 is configured to receive the signals from the one or more environmental sensors 110. Based on the received signals, the processor 120 is further configured to determine an efficacy of the wash cycle; determine an efficacy of the thermal disinfection cycle; and determine the efficacy of the cleaning cycle based on the efficacy of the wash cycle and the efficacy of the thermal disinfection cycle.

In some embodiments, the processor 120 is configured to determine the efficacy of the wash cycle by the steps of: generating temperature data based on the received signals recorded during the wash cycle; selecting a first time period within the wash cycle during which the temperature data exceeds a wash threshold temperature (i.e., 35°C), computing a first set of temperature differentials ($\Delta T_1$) based on the temperature data collected during the first time period and a wash reference temperature (i.e., 40°C); and determining the efficacy of the wash cycle based on a duration of the first time period ($\Delta t_1$), or referred to as the second duration, and the first set of temperature differentials ($\Delta T_1$).

In some embodiments, the first time period is selected by determining a start time of the first time period when the temperature data exceeds the wash threshold temperature and determining an end time of the first time period when the temperature data is below the wash threshold temperature. In some cases, the end time of the first time period can be determined when the temperature data is below the wash threshold temperature for a predetermined duration, for example, for 5 seconds. In some other cases, if the temperature of a wash cycle should be maintained within a certain temperature range (i.e., 30°C - 65°C), the end time of the first time period can be determined as the last time that the temperature data drops below the wash threshold temperature before the temperature data climbs up and above the certain temperature range (i.e., greater than 65°C). In some implementations, the efficacy of the wash cycle ($E_w$) can be proportional to both the sum of ten raised to the power of a predetermined fraction of $\Delta T_1$ (i.e., $\sum 10^{\Delta T_1/10}$) and the first duration ($\Delta t_1$). In some particular embodiments, the efficacy of the wash cycle ($W_E$) can be calculated using equation (1), where T denotes temperature data collected during the first time period, $W_R$ denotes the wash reference temperature, and k is a predetermined number.

$$W_E = \sum 10^{(T-W_R)/k} \times \Delta t_1 \qquad (1)$$

In some embodiments, the processor 120 is further configured to determine the efficacy of the thermal disinfection cycle by the steps of: generating temperature data based on the received signals; selecting a second time period within the thermal disinfection cycle during which the temperature data exceeds a disinfection threshold temperature(i.e., 65°C); computing a second set of temperature differentials ($\Delta T_2$) based on the temperature data collected during the second time period and a disinfection reference temperature(i.e., 80°C); and determine the efficacy of the thermal disinfection cycle based on a duration of the second time period ($\Delta t_2$), or referred to as the second duration, and the second set of temperature differentials ($\Delta T_2$). In some embodiments, the second time period is selected by determining a start time of the second time period when the temperature data exceeds the disinfection threshold temperature and determining an end time of the second time period when the temperature data is below the disinfection threshold temperature. In some cases, the end time of the second time period can be determined when the temperature data is below the disinfection threshold temperature for a predetermined duration of time, for example, 5 seconds. In some implementations, the efficacy of the thermal disinfection cycle ($D_E$) can be proportional to both the sum of ten raised to the power of a predetermined fraction of $\Delta T_2$ (i.e., $\sum 10^{\Delta T2/10}$) and the second duration ($\Delta t_2$). In some particular embodiments, the efficacy of the thermal disinfection cycle ($D_E$) can be calculated using equation (2), where T denotes temperature data collected during the second time period, $D_R$ denotes the disinfection reference temperature, and k is a predetermined number.

$$D_E = \sum 10^{(T-D_R)/k} \times \Delta t_2 \qquad (2)$$

In some embodiments, if the efficacy of the wash cycle is greater than or equal to a wash efficacy threshold and the efficacy of the thermal disinfection cycle is greater than or equal to a disinfection efficacy threshold, the processor 120 can generate a "Pass" signal indicating a satisfactory cleaning cycle. The wash efficacy threshold and the disinfection efficacy threshold can be selected based on experimental results, standard of a country or a region, or an international standard (i.e., 3,000 is often used as a disinfection efficacy threshold for device to be used in contact with human skin). If the efficacy of the wash cycle is below the predetermined wash efficacy threshold, and/or the efficacy of the thermal disinfection cycle is below the disinfection efficacy threshold, the processor 120 can generate a "Fail" signal indicating a ineffective cleaning cycle. The "Pass" or "Fail" signal can be received by the indicator 130 that is communicatively coupled to the processor. In some embodiments, the indicator is configured to produce light visible through a transparent part of the housing 140. The indicator is configured to present a visible indication of the efficacy of the cleaning cycle determined by the processor by, for example, a green light indicating "Pass" and a red light indicating "Fail". In some embodiments, the indicator130 can be implemented by any type of presentation devices suitable to be disposed in a portable device, including but not limited to lighting devices, Light Emitting Diode (LED) devices, a small electronic display, or the like.

Figure 2B illustrates an exemplary system diagram of an electronic indicator device 200. In this embodiment, the device 200 can include a power supply unit 210, a processing unit 220, an optional communication unit 230, an optional indicator 240, an optional switch unit 250, an optional data storage unit 260, an optional clock module 270, and a sensor module 280. In some cases, the device 200 can include a housing 290 to host part of or all components. The device 200 may have several states including, for example, a data collection state, a data analysis state, a transmission state, standby state, and the like.

The processing unit 220 may comprise one or more microprocessors, digital signal processors, processors, PICs (Programmable Interface Controllers), microcontrollers, signal processing circuit, or any other form of computing devices or circuitry. The sensor module 280 is communicatively coupled to the processing unit 220. The sensor module 280 can include one or more sensors including, for example, thermal sensors, orientation sensors, chemical sensors, pH (Potential Hydrogen) sensors, water conductivity sensors, and the like. The processing unit 220 receives signals from one or more sensors in the sensor module 280. The received signals may be, for example, digital streaming signals, digital discrete signals, analog streaming signals, or analog values.

The power supply unit 210 may include one or more rechargeable batteries. In some cases, the power supply unit 210 may include one or more disposable batteries. In some cases, the power supply unit 210 may include a dedicated port to connect to an external power source for charging. In certain embodiments, the sensors of the sensor module 280 are rendered inoperable when a connection to an external power source is established.

In some embodiments, the electronic indicator device 200 may include a communication unit 230. The communication unit 230 can be disposed in the housing and configured to transmit and receive signals and data. The communication unit 230 may include electronics to provide one or more of short-range communication interfaces including, for example, local area network (LAN), interfaces conforming to a known communications standard, such as a Bluetooth standard, IEEE 802 standards (e.g., IEEE 802.11), a ZigBee or similar specification, such as those based on the IEEE 802.15.4 standard, or other public or proprietary wireless protocol. The communication unit 230 may also include electronics to provide one or more of long-range communication interfaces including, for example, wide area network (WAN), cellular network interfaces, satellite communication interfaces, or the like. In some cases, the electronic indicator device 200 may receive commands via the communication unit 230 and modify the configuration or state of the device 200. In some embodiments, the electronic indicator device 200 can include a switch unit 250 configured to change the state of the device 200. The switch unit 250 can be disposed in the housing 290. The switch unit 250 can include, for example, gravity switch, ball switches, mercury switch and the like.

In some embodiments, the electronic indicator device 200 may include a data storage unit 260. The data storage unit 260 is configured to provide storage for signals generated by the sensor module 280 and/or processed data generated by the processing unit 220. The data storage unit 260 can include RAM (random access memory), flash memory, dynamic RAM, static RAM, and the like. In some other embodiments, the electronic indicator device 200 may include a clock module 270 that can be used to regulate sampling rate, provide time stamp for signals generated by the sensor module 280, and/or provide other timing needs. The clock module 270 can include, for example, an oscillator, a crystal oscillator, and the like.

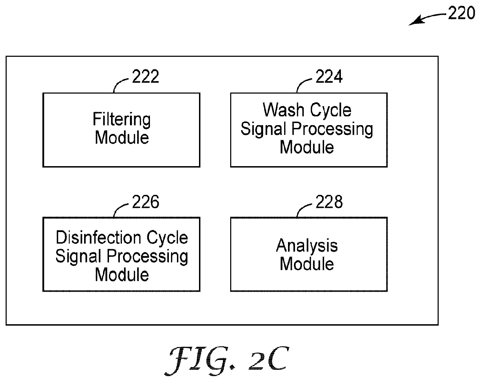

Figure 2C illustrates some exemplary functional modules that may be included in a processing unit 220. These functional modules can be implemented by software or firmware executing on a processor, an analog or digital circuit, or a combination thereof. In some cases, the processing unit 220 may have a filtering module 222 to perform analog and/or digital filtering to enhance the quality of signals received from the sensor module. In some cases, the processing unit 220 may include a wash cycle signal processing module 224 to determine the efficacy of the wash cycle based on the received signals or signals filtered by the filtering module 222, where the signals are generated by the sensor module and related to environmental conditions during the wash cycle. In some cases, the processing unit 220 may include a disinfection cycle signal processing module 226 to determine the efficacy of the thermal disinfection cycle based on the received signals or signals filtered by the filtering module 222, where the signals are generated by the sensor module and related to environmental conditions during the thermal disinfection cycle. In some cases, the processing unit 220 may include an analysis module 228 to determine the overall efficacy of a cleaning cycle based on the computation results of the wash cycle signal processing module 224 and disinfection cycle signal processing module 226.

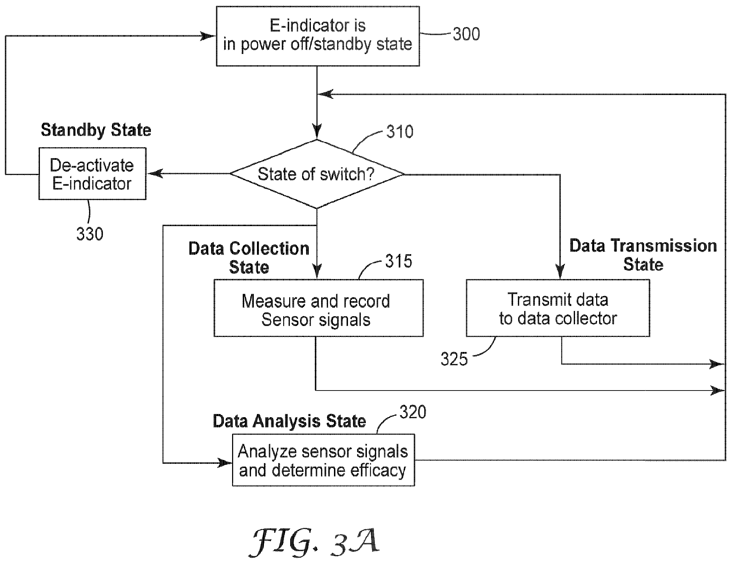

Figure 3A illustrates an exemplary flow diagram of an electronic indicator device (E-indicator). Initially, the E-indicator is in power-off state or standby state (step 300). Next, the E-indicator is activated by a change of the state of switch (i.e., the collective state indicated by switch(es) in the switch unit). The E-indicator evaluates the state of the switch (step 310). If the switch is in the Data Collection State, the E-indicator starts to measure and record sensor signals (step 315). In the Data Collection State, the E-indicator is typically placed in a wash chamber in a washer-disinfector going through a cleaning cycle. If the switch is in the Data Analysis State, the E-indicator starts to analyze the recorded sensor signals and determine the efficacy of the cleaning cycle (step 320). In some implementations, the E-indicator is in the Data Analysis State after the cleaning cycle is completed and sensor signals of a complete cleaning cycle are recorded. In some other cases, the E-indicator can analyze data after sensor signals are measured and recorded during the data collection process (i.e., before the completion of the data collection process) while the E-indicator is in the Data Collection State. If the switch is in Data Transmission State, the E-indicator starts to transmit data to a data collector (step 325). A data collector may be a standalone receiving device with a data repository. A data collector may also be a server that is configured to receive data, analyze data, and store data to a data repository.

A cleaning cycle in a washer-disinfector can include a wash cycle, a rinse cycle, a thermal disinfection cycle, and a drying cycle. In determining an efficacy of a cleaning cycle, it is important to have an effective wash cycle and an effective disinfection cycle. Because the temperature during the rinse cycle is lower than the temperature during the wash cycle and the temperature during disinfection cycle, the end of the wash cycle can be determined as the time when the collected temperature signals are lower than a rinse threshold temperature (i.e., 30°C), or when the collected temperature signals are lower than a rinse threshold temperature (i.e., 30°C) for a certain period of time (i.e., 5 seconds). Because the efficacy of a wash cycle are closely related to the heat input during the wash cycle, the efficacy of the wash cycle can be determined based on the temperature data collected during the wash cycle. Similarly, because the efficacy of a thermal disinfection cycle are closely related to the heat input during the thermal disinfection cycle, the efficacy of the thermal disinfection cycle can be determined based on the temperature data collected during the thermal disinfection cycle.

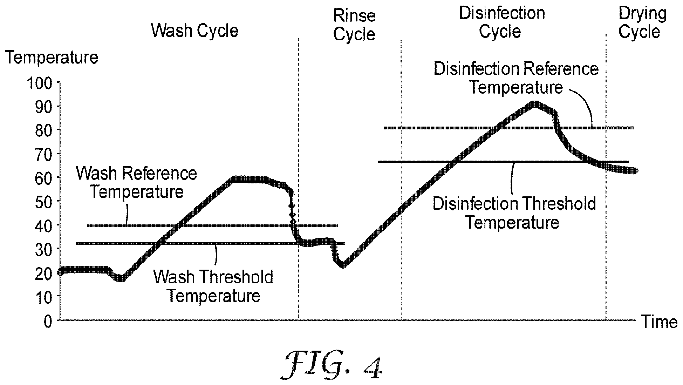

In some embodiments, a method of evaluating an efficacy of a cleaning cycle includes the steps of: receiving temperature data collected during the wash cycle; selecting, by a processing unit, a first time period within the wash cycle during which the temperature data exceeds a wash threshold temperature; determining, by the processing unit, a wash efficacy based on a first set of temperature differentials between the temperature data collected during the first time period and a wash reference temperature; receiving temperature data collected during the thermal disinfection cycle; selecting, by the processing unit, a second time period within the thermal disinfection cycle during which the temperature data exceeds a disinfection temperature threshold; determining, by the processing unit, a disinfection efficacy based on a second set of temperature differentials between the temperature data collected during the second time period and a disinfection reference temperature; and determining, by the processing unit, the efficacy of the cleaning cycle based on the wash efficacy and the disinfection efficacy. Figure 4 is a graph illustrating a time-temperature curve corresponding to a cleaning cycle including a wash cycle, a rinse cycle, a disinfection cycle, and a drying cycle. In some embodiments as illustrated, a wash threshold temperature can be lower than a wash reference temperature and a disinfection threshold temperature can be lower than a disinfection reference temperature.

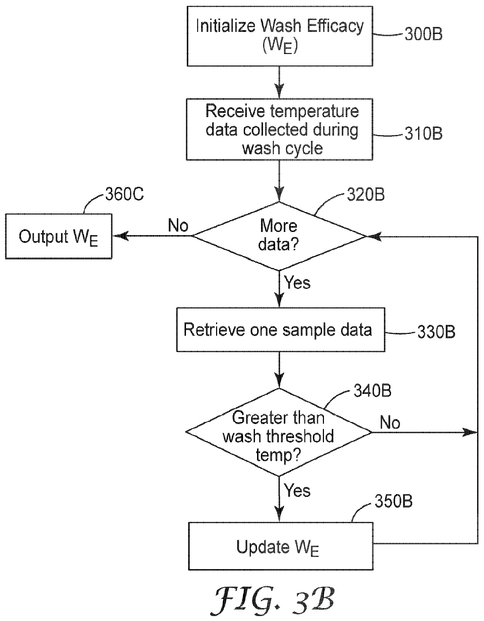

Figure 3B illustrates an exemplary flow diagram for determining an efficacy of a wash cycle. In some embodiments, the flow diagram illustrated in Figure 3B can be implemented by the wash cycle signal processing module 224 in Figure 2C. First, initialize a Wash Efficacy ($W_E$) variable that is used to store the heat equivalence value of the wash cycle (step 300B). Next, receive the temperature data collected during the wash cycle (step 310B). The temperature data can be collected using various sample rates, for example, one temperature data recorded every 0.5 second. Check whether there is more temperature data for the wash cycle (step 320B), if there is, retrieve one sample data (i.e., one sampled temperature data) in accordance with time sequence (step 330B). Compare the sample data with the wash threshold temperature (step 340B), if it is greater than the wash threshold temperature, update $W_E$ (step 350B). If there is no more sample data to be analyzed, output $W_E$ (step 360C). In determining whether the wash cycle is effective, $W_E$ can be compared to a predetermined wash efficacy threshold (i.e., 20,000): if $W_E$ is greater than or equal to the predetermined wash efficacy threshold, the wash cycle passes; and if $W_E$ is lower than the predetermined wash efficacy threshold, the wash cycle fails.

In some embodiments, a time period (*Period W*) in the wash cycle can be selected with the start time as the time when the temperature data exceeds the wash threshold temperature and the end time as the time when the temperature data is below the wash threshold temperature for at least a certain time period (i.e., 5 seconds; 10 seconds, etc.). In some other embodiments, the time period (*Period W*) in the wash cycle can be selected as a time period during which at least 90% of the temperature data exceeds the wash threshold temperature. In these embodiments, the efficacy of the wash cycle (wash efficacy) can be determined based on the temperature signals collected by the sensor unit during *Period W*. In some cases, the efficacy of the wash cycle can be determined to be in a linear relationship with the duration ($\Delta t_1$) of *Period W*. In some other cases, the wash efficacy can be determined based on the duration ($\Delta t_1$), the temperature data collected during *Period W*, and a wash reference temperature. In some embodiments, a first set of temperature differentials ($\Delta T_1$) of the temperature data collected in *Period W* from the wash reference temperature in the unit of Celsius can be computed, and the efficacy of the wash cycle can be determined to be proportional to $\sum 10^{\Delta T_1/10}$.

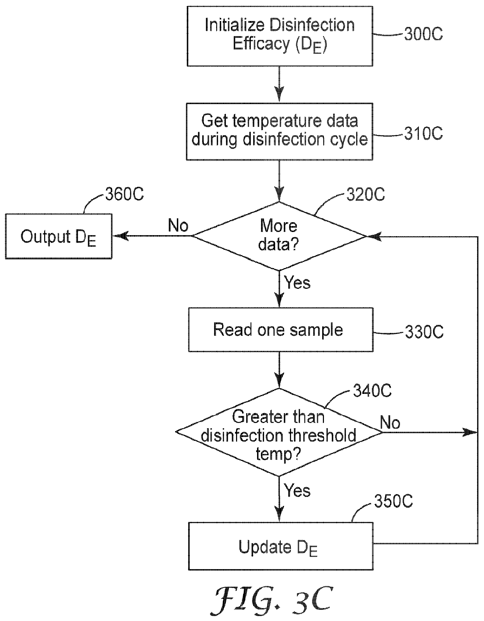

Figure 3C illustrates an exemplary flow diagram for determining an efficacy of a thermal disinfection cycle. In some embodiments, the flow diagram illustrated in Figure 3C can be implemented by the disinfection cycle signal processing module 226 in Figure 2C. First, initialize the Disinfection Efficacy ($D_E$) variable that is used to store the heat equivalence value of the thermal disinfection cycle (step 300C). Next, get the temperature data collected during the thermal disinfection cycle (step 310C). Check whether there is more temperature data for the thermal disinfection cycle (step 320C), if there is, retrieve one sample data (i.e., one sampled temperature data) in accordance with time sequence (step 330C). Compare the sample data with the disinfection threshold temperature (i.e., 65°C) (step 340C), if it is greater than the disinfection threshold temperature, update $D_E$ (step 350C). If there is no more sample data to be analyzed, output $D_E$ (step 360C). In determining whether the thermal disinfection cycle is effective, $D_E$ can be compared to a predetermined disinfection efficacy threshold (i.e., 3,000): if $D_E$ is greater than or equal to the predetermined disinfection efficacy threshold, the thermal disinfection cycle passes; and if $D_E$ is lower than the predetermined disinfection efficacy threshold, the thermal disinfection cycle fails.

In some embodiments, a time period (*Period D*) in the disinfection cycle can be selected with the start time as the time when the temperature data exceeds the disinfection threshold temperature and the end time as the time when the temperature data is below the disinfection threshold temperature for at least a certain time period (i.e., 5 seconds; 10 seconds, etc.). In some embodiments, the time period (*Period D*) in the disinfection cycle can be selected as a time period during which at least 90% of the temperature data exceeds the disinfection threshold temperature. In these embodiments, the efficacy of the thermal disinfection cycle (disinfection efficacy) can be determined based on the temperature signals collected by the sensor unit during *Period D*. In some cases, the disinfection efficacy can be determined to be in a linear relationship with the duration ($\Delta t_2$) of *Period D*. In some other cases, the disinfection efficacy can be determined based on the duration ($\Delta t_2$), the temperature data collected during *Period D*, and a disinfection reference temperature. In some embodiments, a first set of temperature differentials ($\Delta T_2$) of the temperature data collected in *Period D* from the disinfection reference temperature in the unit of Celsius can be computed, and the disinfection efficacy can be determined to be proportional to $\sum 10^{\Delta T_2/10}$. The efficacy of the cleaning cycle can be determined based on the wash efficacy and the disinfection efficacy. In some cases, the cleaning cycle can be determined to be effective if the wash efficacy is greater than a predetermined wash efficacy threshold and the disinfection efficacy is greater than a predetermined disinfection efficacy.

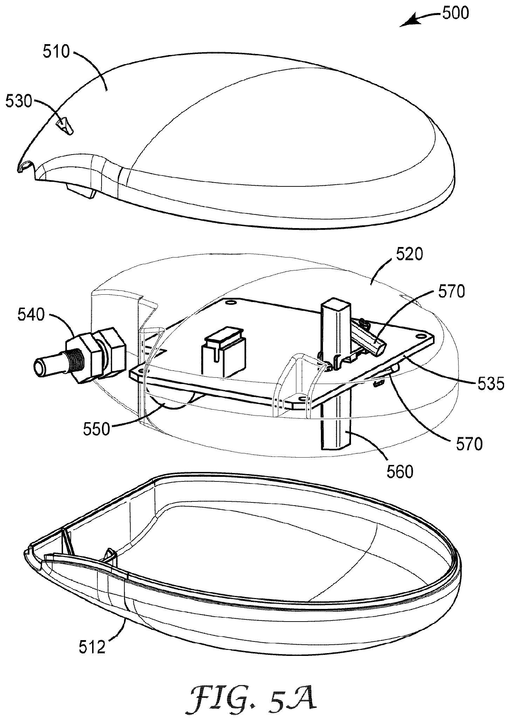

Figure 5A is an exploded view of an exemplary embodiment of an electronic indicator device 500. In this embodiment, the device 500 includes an outer housing including an upper cover 510 and a lower cover 512, an inner housing 520, a fluid port 530, a circuit board 535, a thermal sensor 540, a battery 550, a light guide 560, and a switch 570. In some implementations, the fluid port 530 can be disposed on one or both of the outer housing cover (510 and/or 512) and configured to allow a large surface area of the thermal sensor 540 to be in contact with the fluid. In some cases, the fluid port 530 is designed to have a relative small size (i.e., less than 2 cm long and 0.5 cm wide), as illustrated in Figure 5A. In some other cases, the fluid port 530 can have more than one hole, where each hole can be generally round shape and have a diameter less than 5 mm.

In some implementations, the inner housing 520 can be implemented by hot melt layer, asbestors, foam, glass beads, or the like, to provide adequate heat insulation, pressure-withstood, and waterproof housing. (i.e., 0.5mm - 2mm) In some other implementations, the inner housing 520 can include more than one hot melt layers to provide relative high heat resistance. The hot melt layers can use hot melt materials include, but are not limited to, polyamides, polyurethanes, copolymers of ethylene and vinyl acetate, and olefin polymers modified with more polar species such as maleic anhydride or polyethylene alpha-olefin polymer.

In some embodiments, the circuit board 535 is sealed within the inner housing 520 and hosts the processing unit that can receive signals generated by the thermal sensor 540, optionally process the received signals and/or analyze the received signals to determine the efficacy of a cleaning cycle, and optionally store the received signals and processed data. In some cases, the circuit board 535 can provide electrical contact and communicative contact among various components in the device 500, for example, contact among the thermal sensor 540, the battery 550, and the switch 570. In some implementations, the light guide 560 can be a transparent material allowing light emitting from a light source (not shown in Figure 5A) to be visible at one or both of the outer housing covers (510 and/or 512). The battery 550 can provide power supply to the circuit board 535 and the thermal sensor 540. The switch 570 is configured to change the state of the electronic indicator device 500. In some implementations as illustrated in Figure 5A, the circuit board 535, the battery 550, the light guide 560, and the switch 570 are encapsulated in the inner housing 520.

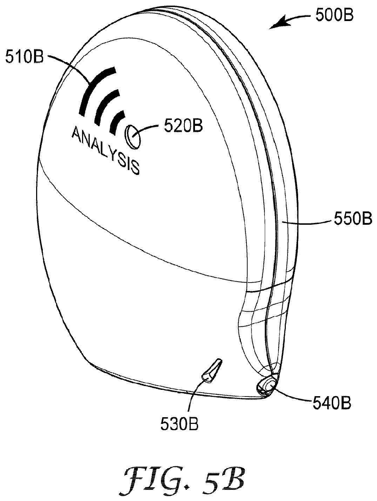

Figure 5B is a perspective view of an embodiment of an electronic indicator device 500B. In the illustrated embodiment, the device 500B includes a label 510B, an indicator 520B, a fluid port 530B, a thermal sensor 540B, and an outer housing 550B. The label 510B can be used to indicate the state of the device 500B, which is described in details below. The thermal sensor 540B can be any type of thermal sensor or temperature sensor capable of detecting temperature of its surrounding environment including, for example, platinum rhodium sensor. The indicator 520B can be any type of light source with optional light guide that is visible from the outer housing 550B. The indicator 520B can provide the status of the cleaning monitoring, for example, a flashing light indicating data collection in progress, a light-off indicating a standby state, a green light indicating a "Pass" cleaning cycle, and a red light indicating a "Fail" cleaning cycle.

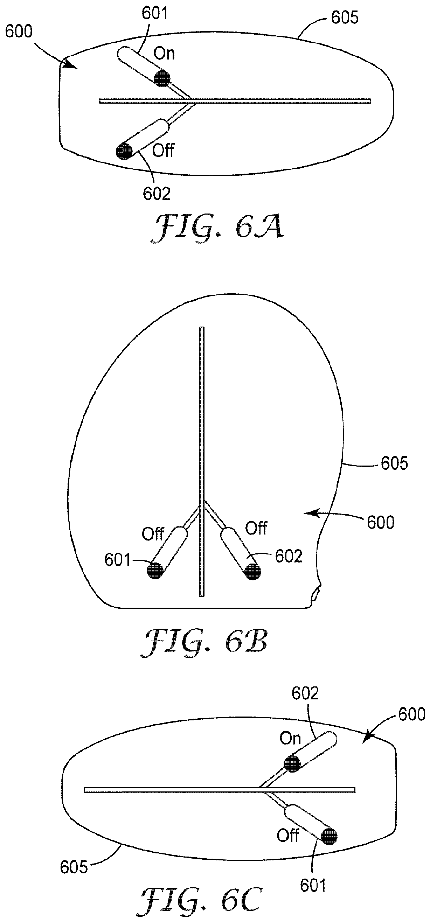

Figures 6A, 6B, and 6C illustrate a cross-sectional view of an exemplary embodiment of a switch 600 used by an electronic indicator device 605, where the switch 600 presents three different states in the three figures. In the illustrated embodiment, the switch 600 can include ball switch 601 and ball switch 602. Ball switch 601 and ball switch 602 can have different states depending on the position and orientation of the device 605. For example, in Figure 6A, ball switch 601 is "On" and ball switch 602 is "Off" while the device 605 is horizontally placed with one side up. The processing unit of the device 605 can take the state of ball switch 600 as an input to alternate the state of the device 605. For example, the device 605 can be in data collection state when ball switch 601 is "On" and ball switch 602 is "Off" (Figure 6A); in data analysis state/standby state when ball switch 601 and ball switch 602 are both off (Figure 6B); and in data transmission state when ball switch 601 is "Off' and ball switch 602 is "On" (Figure 6C).

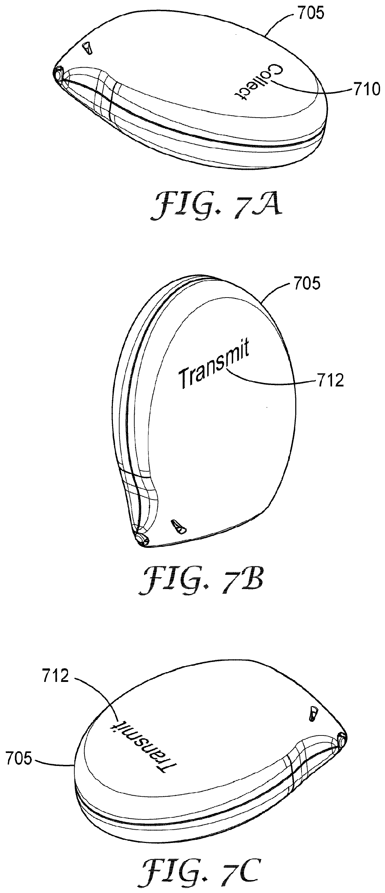

Figures 7A, 7B, 7C illustrate an electronic indicator device 705 with labels corresponding to the electronic indicator device 605 with its respective state as illustrated in Figures 6A, 6B, and 6C. The labels on the electronic indicator device 705 can be used to indicate the state of the device 705. In Figure 7A, the device 705 horizontally placed with the label 710 "collect" facing upward can indicate the device 705 in the Data Collection State. Similarly, in Figure 7C, the device 705 horizontally placed with the label 712 "transmit" facing upward can indicate the device 705 in the Data Transmission State. In Figure 7B, the device 705 is vertically placed and in data analysis state /standby state. In the illustrated embodiment, the electronic indicator device 705 can be placed horizontally with the label 710 "collect" facing upward in a wash chamber of a washer-disinfector during a cleaning cycle. After the cleaning cycle completes, the electronic indicator device 705 can be taken out of the wash chamber and placed vertically to start the data analysis process. After the electronic indicator device 705 shows an indication of "Pass" or "Fail", the device 705 can be placed horizontally with the label 712 "transmit" facing upward to transmit data; or the device 705 can change to a standby state after it has provided "Pass" or "Fail" indication in the vertical position for a predetermined period time, for example, two minutes Alternatively, the device 705 can be change to the standby state after the device 705 has completed data transmission.

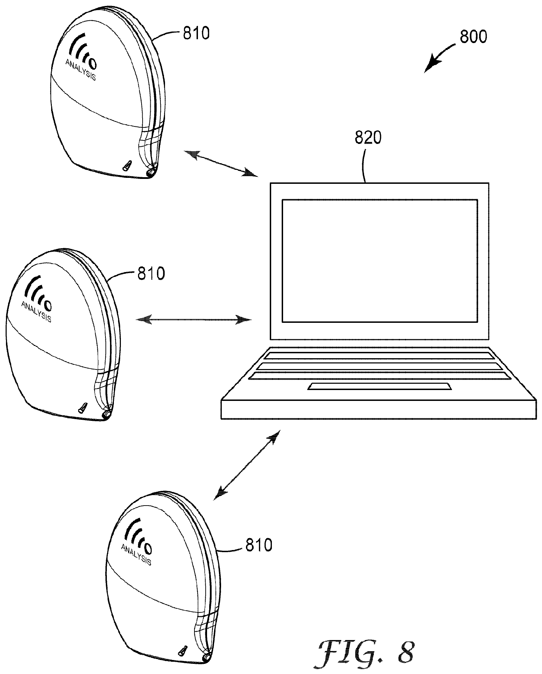

In some embodiments, one or more electronic indicator devices can be used in a cleaning monitoring system to monitor efficacy of washer-disinfectors. Figure 8 illustrates a system diagram of an exemplary embodiment of a cleaning monitoring system 800. The cleaning monitoring system 800 includes several electrical indicator devices 810 and a monitoring server 820. The electronic indicator devices 810 are configured to be placed within wash chambers and to monitor efficacy of cleaning cycles, where each cleaning cycle includes a wash cycle and a thermal disinfection cycle. In some cases, an electronic indictor device 810 includes a thermal sensor configured to detect temperature in the wash chamber and generate signals related to the temperature. In some other cases, the electronic indicator device 810 may include a processor communicatively coupled to the thermal sensor and configured to receive signals from the thermal sensor and determine the efficacy of a cleaning cycle based on the received signals. In some cases, the electronic indicator device 810 may further include a wireless transceiver communicatively coupled to the processor and/or the thermal sensor and capable of transmitting signals and data to the monitoring server 820 and receiving commands from the monitoring server 820. In some embodiments, the electronic indicator device 810 may transmit signals collected by the thermal sensor and/or processed data by the processor via the wireless transceiver. In some cases, the electrical indicator device 810 may include an indicator communicatively coupled to the processor and configured to present the efficacy of the cleaning cycle determined by the processor.

In some implementations, the electronic indicator device 810 can start recording data when the state of the device 810 is changed to "data collection state." The device 810 may optionally include a switch that can change its state based on the orientation and/or position of the device 810 The device 810 may further include labels indicating the state of the device 810 when the device is at certain position and/or orientation. For example, the switch state can be in "data collection state" when the device is horizontally laying with the side having a label indicating data collection state (i.e., "collect", etc.) facing upward, in "data transmission state" when the device is horizontally laying with the side having a label indicating data transmission state (i.e., "transmit", etc.) facing upward, and in "data analysis state" or "standby state" when the device is vertically placed. In some embodiments, the state of the device 810 may also be changed in response to a command sent from the monitoring server 820.

In some embodiments, the monitoring server 820 can include a wireless transceiver configured to receive signals and data sent from the one or more electronic indicator devices 810. The monitoring server 820 may include a data storage unit configured to store the received signals and data. In some embodiments, however, the electronic indictor device 810 does not include a processor and temperature data are transmitted to the monitoring server 820 for analysis. In such embodiments, after receiving signals collected during a cleaning cycle including a wash cycle and a thermal disinfection cycle, the monitoring server 820 can include a processing unit to determine an efficacy of the wash cycle ($W_E$) using a process similar to the one illustrated in Figure 3B, determine an efficacy of the thermal disinfection cycle ($D_E$) using a process similar to the one illustrated in Figure 3C, and then determine an efficacy of the cleaning cycle based on $W_E$ and $D_E$.

## Examples

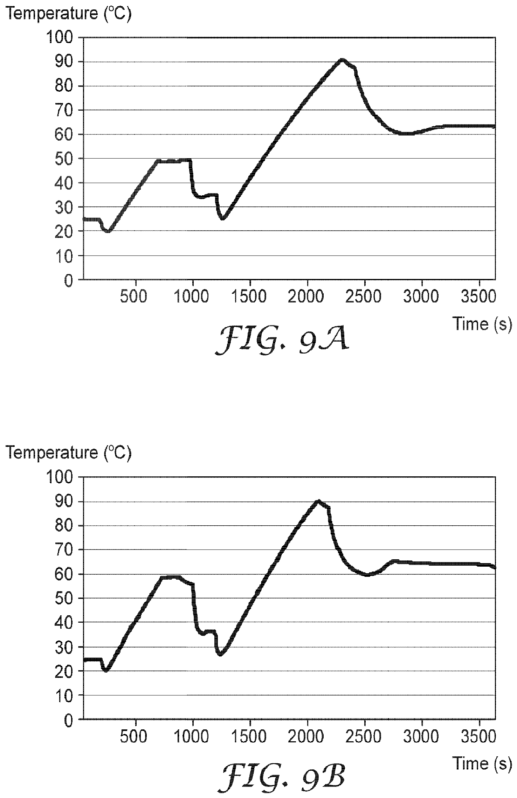

Example 1: Use a Getinge 46 washer-disinfector. Use enzyme cleaner protease with 0.25% concentration and 400 dilution rate. Use TOSI (Test Object Surgical Instrument) to verify the cleaning efficiency. Place the electronic indicator device with instruments to be cleaned and 10 TOSIs in the wash chamber of the washer-disinfector before a cleaning cycle. The 10 TOSIs are placed in different stands in the wash chamber. Run the wash cycle at 50°C for 5 minutes with enzyme cleaner and the thermal disinfection cycle at 90°C for 1 minute. The temperature data collected by the electronic indicator device is illustrated in Figure 9A. Computed result of the wash efficacy using equation (1), with $W_R$ = 40°C and k = 10, is $W_E$ = 16783. Computed result of the disinfection efficacy using equation (2) ($D_R$ = 80°C and k = 10) is $D_E$ = 2797.54. 10% TOSIs (i.e., 1 TOSI) indicators showed inadequate cleaning efficiency.

Example 2: Use a Getinge 46 washer-disinfector. Use enzyme cleaner protease with 0.25% concentration and 400 dilution rate. Use TOSI (Test Object Surgical Instrument) to verify the cleaning efficiency. Place the electronic indicator device with instruments to be cleaned and 10 TOSIs in the wash chamber of the washer-disinfector before a cleaning cycle. The 10 TOSIs are placed in different stands in the wash chamber. Run the wash cycle at 60°C for 1 minute with enzyme cleaner and the thermal disinfection cycle at 90°C for 1 minute. The temperature data collected by the electronic indicator device is illustrated in Figure 9B. Computed result of the wash efficacy using equation (1), with $W_R$ = 40°C and k = 10, is $W_E$ = 28040.76. Computed result of the disinfection efficacy using equation (2) ($D_R$ = 80°C and k = 10) is $D_E$ = 2797.54. All TOSIs (i.e., 1 TOSI) indicators showed adequate cleaning efficiency.

## Claims

1. An apparatus (100) for determining the efficacy of a cleaning cycle that is configured to be placed in a wash chamber containing a fluid, the cleaning cycle comprising a wash cycle and a thermal disinfection cycle, the apparatus (100) comprising:

   a housing (140), the exterior of the housing configured to be in contact with the fluid;
   a thermal sensor (110) configured to provide signals indicative of temperature of the fluid, at least part of the thermal sensor disposed in the housing (140);

   **characterised by**

   a processor (120) disposed in the housing and communicatively coupled to the thermal sensor (110), the processor (120) configured to

      receive the signals from the thermal sensor (110), and based on the received signals:

         determine an efficacy of the wash cycle;
         determine an efficacy of the thermal disinfection cycle; and
         determine the efficacy of the cleaning cycle based on the efficacy of the wash cycle and the efficacy of the thermal disinfection cycle.

**2.** The apparatus (100) of claim 1, wherein the processor (120) is further configured to determine the efficacy of the wash cycle by the steps of:

generating temperature data based on the received signals collected during the wash cycle;
selecting a first time period within the wash cycle during which the temperature data exceeds a wash threshold temperature;
computing a first set of temperature differentials ($\Delta T_1$) based on the temperature data collected during the first time period and a wash reference temperature; and
determining the efficacy of the wash cycle based on a duration of the first time period and the first set of temperature differentials.

**3.** The apparatus (100) of claim 2, wherein the processor (120) is further configured to determine the efficacy of the thermal disinfection cycle by the steps of:

generating temperature data based on the received signals collected during the thermal disinfection cycle;
selecting a second time period within the thermal disinfection cycle during which the temperature data exceeds a disinfection threshold temperature;
computing a second set of temperature differentials ($\Delta T_2$) based on the temperature data collected during the second time period and a disinfection reference temperature; and
determining the efficacy of the thermal disinfection cycle based on a duration of the second time period and the second set of temperature differentials.

**4.** The apparatus (100) of claim 2, wherein the processor (120) is further configured to

determine the efficacy of the wash cycle to be proportional to $\sum 10^{\Delta T_1/k}$, where k is a predetermined number and $\Delta T_1$ is the duration of the first time period.

**5.** The apparatus (100) of claim 3, wherein the processor (120) is further configured to

determine the efficacy of the thermal disinfection cycle to be proportional to $\sum 10^{\Delta T_2/k}$ where k is a predetermined number and $\Delta T_2$ is the duration of the second time period.

**6.** The apparatus (100) of claim 1, further comprising:

an indicator (130) communicatively coupled to the processor (120) and configured to indicate the efficacy of the cleaning cycle determined by the processor (120),
wherein the housing (140) comprises a transparent portion and the indicator (130) is visible through transparent portion of the housing (140).

**7.** The apparatus of claim 1, further comprising:

a communication unit disposed in the housing and configured to transmit the temperature data.

**8.** The apparatus of claim 1, further comprising:

a switch disposed in the housing and configured to change a state of the apparatus.

**9.** The apparatus of claim 1, further comprising:

a fluid port disposed on the housing and configured to allow the thermal sensor to be in contact with the fluid.

**10.** A method of evaluating an efficacy of a cleaning cycle comprising a wash cycle and a thermal disinfection cycle, the method comprising:

receiving temperature data and time data collected during the wash cycle;
selecting, by a processor (120), a first time period within the wash cycle during which the temperature data is indicative of temperatures exceeding a wash threshold temperature;
determining, by the processor (120), a wash efficacy based on the temperature data collected during the first time period;

receiving temperature data and time data collected during the thermal disinfection cycle;

selecting a second time period within the thermal disinfection cycle during which the temperature data is indicative of temperatures exceeding a disinfection temperature threshold;

determining, by the processor (120), a disinfection efficacy based on the temperature data collected during the second time period; and

determining, by the processor (120), the efficacy of the cleaning cycle based on the wash efficacy and the disinfection efficacy.

**11.** The method of claim 10, further comprising:

indicating the efficacy of the cleaning cycle via an indicator (130).

**12.** The method of claim 10, wherein the wash efficacy has a linear relationship to the duration of the first time period.

**13.** The method of claim 10, further comprising:

computing a first set of temperature differentials ($\Delta T_1$) of the temperature data collected in the first time period from a wash reference temperature in the unit of Celsius; and

determining the wash efficacy to be proportional to $\sum 10^{\Delta T_1/10}$.

**14.** The method of claim 10, wherein the disinfection efficacy has a linear relationship to the duration of the second time period.

**15.** The method of claim 10, further comprising:

computing a second set of temperature differentials ($\Delta T_2$) of the temperature data collected in the second time period from a disinfection reference temperature in the unit of Celsius; and

determining the disinfection efficacy to be to be proportional to $\sum 10^{\Delta T_2/10}$.

**16.** The method of claim 10, further comprising:

transmitting, via a communication unit, the temperature data and time data of the wash cycle and the temperature data and time data of the disinfection cycle.


**Patentansprüche**

**1.** Vorrichtung (100) zum Ermitteln der Wirksamkeit eines Reinigungszyklus, der so gestaltet ist, dass er in einer Waschkammer stattfindet, die ein Fluid enthält, wobei der Reinigungszyklus einen Waschzyklus und einen Wärme-Desinfektionszyklus umfasst, wobei die Vorrichtung (100) umfasst:

ein Gehäuse (140), wobei das Äußere des Gehäuses so gestaltet ist, dass es in Kontakt mit dem Fluid steht;

einen Wärmesensor (110), der so gestaltet ist, dass Signale bereitgestellt werden, die eine Temperatur des Fluids anzeigen, wobei mindestens ein Teil des Wärmesensors im Gehäuse (140) angeordnet ist;

**gekennzeichnet durch**

einen Prozessor (120), der im Gehäuse angeordnet und zu Datenaustausch-zwecken mit dem Wärmesensor (110) verbunden ist, wobei der Prozessor (120) so gestaltet ist, dass er die Signale vom Wärmesensor (110) empfängt und auf der Grundlage der empfangenen Signale:

eine Wirksamkeit des Waschzyklus ermittelt;

eine Wirksamkeit des Wärme-Desinfektionszyklus ermittelt; und

die Wirksamkeit des Reinigungszyklus auf der Grundlage der Wirksamkeit des Waschzyklus und des Wärme-Desinfektionszyklus ermittelt.

**2.** Vorrichtung (100) nach Anspruch 1, wobei der Prozessor (120) ferner so gestaltet ist, dass die Wirksamkeit des Waschzyklus ermittelt wird durch die Schritte:

Erzeugen von Temperaturdaten auf der Grundlage der empfangenen Signale, die während des Waschzyklus erfasst wurden;

Auswählen eines ersten Zeitraums innerhalb des Waschzyklus, währenddessen die Temperaturdaten eine Wasch-Schwellenwerttemperatur überschreiten;

Berechnen eines ersten Satzes von Temperaturunterschieden ($\Delta T_1$) auf der Grundlage der während des ersten Zeitraums erfassten Temperaturdaten und einer Wasch-Referenztemperatur; und

Ermitteln der Wirksamkeit des Waschzyklus auf der Grundlage einer Dauer des ersten Zeitraums und des ersten Satzes von Temperaturunterschieden.

3. Vorrichtung (100) nach Anspruch 2, wobei der Prozessor (120) ferner so gestaltet ist, dass die Wirksamkeit des Wärme-Desinfektionszyklus ermittelt wird durch die Schritte:

Erzeugen von Temperaturdaten auf der Grundlage der empfangenen Signale, die während des Wärme-Desinfektionszyklus erfasst wurden;

Auswählen eines zweiten Zeitraums innerhalb des Wärme-Desinfektionszyklus, währenddessen die Temperaturdaten eine Desinfektions-Schwellenwerttemperatur überschreiten;

Berechnen eines zweiten Satzes von Temperaturunterschieden ($\Delta T_2$) auf der Grundlage der während des zweiten Zeitraums erfassten Temperaturdaten und einer Desinfektions-Referenztemperatur; und

Ermitteln der Wirksamkeit des Wärme-Desinfektionszyklus auf der Grundlage einer Dauer des zweiten Zeitraums und des zweiten Satzes von Temperaturunterschieden.

4. Vorrichtung (100) nach Anspruch 2, wobei der Prozessor (120) ferner so gestaltet ist, dass die Wirksamkeit des Waschzyklus als proportional zu $\sum 10^{\Delta T1/k}$ ermittelt wird, wobei k eine vorgegebene Zahl und $\Delta T_1$ die Dauer des ersten Zeitraums ist.

5. Vorrichtung (100) nach Anspruch 3, wobei der Prozessor (120) ferner so gestaltet ist, dass die Wirksamkeit des Wärme-Desinfektionszyklus als proportional zu $\sum 10^{\Delta T2/k}$ ermittelt wird, wobei k eine vorgegebene Zahl und $\Delta T_2$ die Dauer des zweiten Zeitraums ist.

6. Vorrichtung (100) nach Anspruch 1, ferner umfassend:

eine Anzeige (130), die zu Datenaustauschzwecken mit dem Prozessor (120) verbunden und so gestaltet ist, dass die durch den Prozessor (120) ermittelte Wirksamkeit des Reinigungszyklus angezeigt wird, wobei das Gehäuse (140) einen durchsichtigen Abschnitt umfasst und die Anzeige (130) durch den durchsichtigen Abschnitt des Gehäuses (140) sichtbar ist.

7. Vorrichtung nach Anspruch 1, ferner umfassend:

eine Datenaustauscheinheit, die im Gehäuse angeordnet und so gestaltet ist, dass die Temperaturdaten übertragen werden.

8. Vorrichtung nach Anspruch 1, ferner umfassend:

einen im Gehäuse angeordneten Schalter, der so gestaltet ist, dass ein Zustand der Vorrichtung geändert werden kann.

9. Vorrichtung nach Anspruch 1, ferner umfassend:

eine Fluidöffnung, die am Gehäuse angeordnet und so gestaltet ist, dass der Wärmesensor mit dem Fluid in Kontakt stehen kann.

10. Verfahren zum Bewerten einer Wirksamkeit eines Reinigungszyklus, der einen Waschzyklus und einen Wärme-Desinfektionszyklus umfasst, wobei das Verfahren umfasst:

Empfangen von Temperaturdaten und Zeitdaten, die während des Waschzyklus erfasst werden;
durch einen Prozessor (120) Auswählen eines ersten Zeitraums innerhalb des Waschzyklus, währenddessen die Temperaturdaten Temperaturen anzeigen, die eine Wasch-Schwellenwerttemperatur überschreiten;
durch den Prozessor (120) Ermitteln einer Waschwirksamkeit auf der Grundlage der Temperaturdaten, die während des ersten Zeitraums empfangen wurden;
Empfangen von Temperaturdaten und Zeitdaten, die während des Wärme-Desinfektionszyklus erfasst werden;
Auswählen eines zweiten Zeitraums innerhalb des Wärme-Desinfektionszyklus, währenddessen die Temperaturdaten Temperaturen anzeigen, die einen Desinfektions-Temperaturschwellenwert überschreiten;
durch den Prozessor (120) Ermitteln einer Desinfektionswirksamkeit auf der Grundlage der Temperaturdaten, die während des zweiten Zeitraums empfangen wurden;
durch den Prozessor (120) Ermitteln der Wirksamkeit des Reinigungszyklus auf der Grundlage der Waschwirksamkeit und der Desinfektionswirksamkeit.

**11.** Verfahren nach Anspruch 10, ferner umfassend:

ein Anzeigen der Wirksamkeit des Reinigungszyklus über eine Anzeige (130).

**12.** Verfahren nach Anspruch 10, wobei die Waschwirksamkeit ein lineares Verhältnis zur Dauer des ersten Zeitraums aufweist.

**13.** Verfahren nach Anspruch 10, ferner umfassend:

Berechnen eines ersten Satzes von Temperaturunterschieden ($\Delta T_1$) der im ersten Zeitraum erfassten Temperaturdaten von einer Wasch-Referenztemperatur in der Maßeinheit Grad Celsius; und Ermitteln der Waschwirksamkeit als proportional zu $\sum 10^{\Delta T1/10}$.

**14.** Verfahren nach Anspruch 10, wobei die Desinfektionswirksamkeit ein lineares Verhältnis zur Dauer des zweiten Zeitraums aufweist.

**15.** Verfahren nach Anspruch 10, ferner umfassend:

Berechnen eines zweiten Satzes von Temperaturunterschieden ($\Delta T_2$) der im Zeitraum erfassten Temperaturdaten von einer Desinfektions-Referenztemperatur in der Maßeinheit Grad Celsius; und
Ermitteln der Desinfektionswirksamkeit als proportional zu $\sum 10^{\Delta T2/10}$.

**16.** Verfahren nach Anspruch 10, ferner umfassend:

Übertragen der Temperaturdaten und Zeitdaten des Waschzyklus und der Temperaturdaten und Zeitdaten des Desinfektionszyklus über eine Datenaustauscheinheit.

## Revendications

**1.** Appareil (100) pour déterminer l'efficacité d'un cycle de nettoyage, qui est conçu pour être placé dans une chambre de lavage contenant un fluide, le cycle de nettoyage comprenant un cycle de lavage et un cycle de désinfection thermique, l'appareil (100) comprenant :

un logement (140), l'extérieur du logement étant conçu pour être en contact avec le fluide ;
un capteur thermique (110) conçu pour fournir des signaux indiquant la température du fluide, au moins une partie du capteur thermique étant disposée dans le logement (140) ;
**caractérisé par**
un processeur (120) disposé dans le logement et couplé par communications avec le capteur thermique (110), le processeur (120) étant conçu pour recevoir les signaux provenant du capteur thermique (110), et sur base des signaux reçus :

déterminer une efficacité du cycle de lavage ;
déterminer une efficacité du cycle de désinfection thermique ; et
déterminer l'efficacité du cycle de nettoyage sur a base de l'efficacité du cycle de lavage et de l'efficacité du cycle de désinfection thermique.

**2.** Appareil (100) selon la revendication 1, dans lequel le processeur (120) est en outre conçu pour déterminer l'efficacité du cycle de lavage par les étapes consistant à :

générer des données de température sur base des signaux reçus recueillis pendant le cycle de lavage ;

sélectionner une première période de temps au cours du cycle de lavage pendant laquelle les données de température dépassent une température seuil de lavage ;

calculer un premier ensemble de différenciels de température ($\Delta T_1$) sur la base des données de température recueillies pendant la première période de temps et d'une température de référence de lavage ; et

déterminer l'efficacité du cycle de lavage sur la base d'une durée de la première période de temps et du premier ensemble de différenciels de température.

3. Appareil (100) selon la revendication 2, dans lequel le processeur (120) est en outre conçu pour déterminer l'efficacité du cycle de désinfection thermique par les étapes consistant à :

générer des données de température sur la base des signaux reçus recueillis pendant le cycle de désinfection thermique ;

sélectionner une deuxième période de temps au cours du cycle de désinfection thermique pendant laquelle les données de température dépassent une température seuil de désinfection ;

calculer un deuxième ensemble de différenciels de température ($\Delta T_2$) sur la base des données de température recueillies pendant la deuxième période de temps et d'une température de référence de désinfection ; et

déterminer l'efficacité du cycle de désinfection thermique sur la base d'une durée de la deuxième période de temps et du deuxième ensemble de différenciels de température.

4. Appareil (100) selon la revendication 2, dans lequel le processeur (120) est en outre conçu pour déterminer l'efficacité du cycle de lavage comme étant proportionnelle à $\sum 10^{\Delta T1/k}$, où k est un nombre prédéterminé et $\Delta T_1$ la durée de la première période de temps.

5. Appareil (100) selon la revendication 3, dans lequel le processeur (120) est en outre conçu pour déterminer l'efficacité du cycle de désinfection thermique comme étant proportionnelle à $\sum 10^{\Delta T2/k}$ où k est un nombre prédéterminé et $\Delta T_2$ la durée de la deuxième période de temps.

6. Appareil (100) selon la revendication 1, comprenant en outre :

un indicateur (130) couplé par communications avec le processeur (120) et conçu pour indiquer l'efficacité du cycle de nettoyage déterminée par le processeur (120),

dans lequel le logement (140) comprend une partie transparente et l'indicateur (130) est visible à travers la partie transparente du logement (140).

7. Appareil selon la revendication 1, comprenant en outre :

une unité de communication disposée dans le logement et conçue pour transmettre les données de température.

8. Appareil selon la revendication 1, comprenant en outre :

un commutateur disposé dans le logement et conçu pour changer un état de l'appareil.

9. Appareil selon la revendication 1, comprenant en outre :

un orifice de fluide disposé sur le logement et conçu pour permettre au capteur thermique d'être en contact avec le fluide.

10. Procédé d'évaluation de l'efficacité d'un cycle de nettoyage comprenant un cycle de lavage et un cycle de désinfection thermique, le procédé comprenant :

la réception de données de température et de données de temps recueillies pendant le cycle de lavage ;

la sélection, par un processeur (120), d'une première période de temps au cours du cycle de lavage pendant laquelle les données de température sont indicatives de températures dépassant une température seuil de lavage ;

la détermination, par le processeur (120), d'une efficacité de lavage sur base des données de température recueillies pendant la première période de temps ;

la réception de données de température et de données de temps recueillies pendant le cycle de désinfection thermique ;

la sélection d'une deuxième période de temps au cours du cycle de désinfection thermique pendant laquelle les données de température sont indicatives de températures dépassant un seuil de température de désinfection ;

la détermination, par le processeur (120), d'une efficacité de désinfection sur la base des données de température recueillies pendant la deuxième période de temps ; et

la détermination, par le processeur (120), de l'efficacité du cycle de nettoyage sur la base de l'efficacité de lavage et de l'efficacité de désinfection.

11. Procédé selon la revendication 10, comprenant en outre :

l'indication de l'efficacité du cycle de nettoyage par l'intermédiaire d'un indicateur (130).

12. Procédé selon la revendication 10, dans lequel l'efficacité de lavage présente une relation linéaire avec la durée de la première période de temps.

13. Procédé selon la revendication 10, comprenant en outre :

le calcul d'un premier ensemble de différenciels de température ($\Delta T_1$) des données de température recueillies dans la première période de temps par rapport à une température de référence de lavage en degrés Celsius ; et la détermination de l'efficacité de lavage comme étant proportionnelle à $\sum 10^{\Delta T1/10}$.

14. Procédé selon la revendication 10, dans lequel l'efficacité de désinfection présente une relation linéaire à la durée de la deuxième période de temps.

15. Procédé selon la revendication 10, comprenant en outre :

le calcul d'un deuxième ensemble de différenciels de température ($\Delta T_2$) des données de température recueillies dans la deuxième période de temps par rapport à une température de référence de désinfection en degrés Celsius ; et

la détermination de l'efficacité de désinfection comme étant proportionnelle à $\sum 10^{\Delta T2/10}$.

16. Procédé selon la revendication 10, comprenant en outre :

la transmission, par l'intermédiaire d'une unité de communication, des données de température et des données de temps du cycle de lavage et des données de température et des données de temps du cycle de désinfection.

Enzyme
Activity (% )

$\mathcal{FIG.}$ 1

*FIG. 2A*

*FIG. 2B*

220

222

Filtering
Module

224

Wash Cycle
Signal Processing
Module

226

Disinfection Cycle
Signal Processing
Module

228

Analysis
Module

*FIG. 2C*

EP 2 854 875 B1

```
                    ┌─────────────────────┐
                    │   E-indicator is    │──── 300
                    │ in power off/standby state │
                    └─────────────────────┘
```

**Standby State**

De-activate
E-indicator ──── 330

State of switch? ──── 310

**Data Collection State** ──── 315

Measure and record
Sensor signals

**Data Transmission State**

Transmit data
to data collector ──── 325

**Data Analysis State** ──── 320

Analyze sensor signals
and determine efficacy

FIG. 3A

Initialize Wash Efficacy ($W_E$) —300B

↓

Receive temperature data collected during wash cycle —310B

↓

360C

Output $W_E$ ← No — More data? 320B

↓ Yes

Retrieve one sample data —330B

↓

340B

Greater than wash threshold temp? — No →

↓ Yes

350B

Update $W_E$

*FIG. 3B*

EP 2 854 875 B1

Initialize Disinfection Efficacy ($D_E$) —300C

Get temperature data during disinfection cycle —310C

360C— Output $D_E$

320C

More data?

No

Yes

Read one sample —330C

340C

Greater than disinfection threshold temp?

No

Yes

Update $D_E$ —350C

*FIG. 3C*

FIG. 4

500

510

530

520

570

540

535

550

570

560

512

*FIG. 5A*

**FIG. 5B**

600

601

605

On

602

Off

*FIG. 6A*

605

600

601 Off

Off 602

*FIG. 6B*

602

600

On

605

Off

601

*FIG. 6C*

705

Collect

710

## FIG. 7A

705

Transmit

712

## FIG. 7B

712

Transmit

705

## FIG. 7C

FIG. 8

Temperature (°C)

FIG. 9A

Temperature (°C)

FIG. 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007001866 A2
- EP 0604387 A1
- EP 1308718 A2
- WO 9836781 A1